# EUROPÄISCHE PATENTANMELDUNG

(11) **EP 1 322 143 A2**
(43) Veröffentlichungstag der Anmeldung: **25.06.2003**
(21) Anmeldenummer: 02102808.9
(22) Anmeldetag: 18.12.2002
(51) Int. Cl.: H05G 1/46

(54) **Verfahren und Vorrichtung zur Belichtung von Röntgenaufnahmen**

(30) Priorität: 21.12.2001 DE 10163583
(71) Anmelder: Philips Intellectual Property & Standards GmbH, 20099 Hamburg (DE); Koninklijke Philips Electronics N.V., 5621 BA Eindhoven (NL)
(72) Erfinder: Bothe, Uwe, Postfach 50 05 42 52088, Aachen (DE); Semke, Axel, Postfach 50 04 42 52088, Aachen (DE)
(74) Vertreter: Volmer, Georg, Dipl.-Ing.

(57) **Zusammenfassung**

Es wird ein Verfahren und eine Vorrichtung zur Begrenzung einer in ein Objekt eintretenden Strahlung (Eintritts-Dosisleistung) bei der Bestrahlung des Objektes mittels einer Strahlenquelle (1), insbesondere bei der Belichtung von Röntgenaufnahmen, beschrieben. Dabei werden diejenigen Wertepaare einer Regelkurve, die zu einer Eintritts-Dosisleistung an dem Untersuchungsobjekt führen, die eine vorgegebene maximale Eintritts-Dosisleistung übersteigt, mit einem berechneten Faktor korrigiert. Dadurch entsteht eine korrigierte Regelkurve, die in voller Länge zur Einstellung gewünschter Röhrenparameter (Röhrenspannung, Röhrenstrom oder Ladung) genutzt werden kann, ohne dass die Gefahr eines Überschreitens der maximalen Eintritts-Dosisleistung besteht.

## Beschreibung

Die Erfindung betrifft ein Verfahren und eine Vorrichtung zur Begrenzung einer in ein Objekt eintretenden Strahlung (Eintritts-Dosisleistung) bei der Bestrahlung des Objektes mittels einer Strahlenquelle, insbesondere bei der Belichtung von Röntgenaufnahmen. Die Erfindung betrifft ferner ein Verfahren und eine Vorrichtung zur Belichtung von Röntgenaufnahmen unter Anwendung eines Verfahrens bzw. einer Vorrichtung zur Begrenzung der Eintritts-Dosisleistung. Die Erfindung betrifft auch einen Röntgengenerator zur Durchführung eines dieser Verfahren sowie eine entsprechende Röntgenanlage bzw. ein Röntgensystem mit einer dieser Vorrichtungen.

Röntgenanlagen oder -systeme umfassen im allgemeinen einen oder mehrere Röntgenstrahler mit einer Röntgenröhre zur Erzeugung von Röntgenstrahlen, sowie einen Röntgengenerator mit einer Spannungsversorgung (Hochspannungserzeuger), die ein Netzteil umfasst und die die zum Betrieb der Röntgenröhre erforderliche Hochspannung zur Verfügung stellt. Wenn der Röntgenstrahler und die die Hochspannung erzeugenden Teile zu einer konstruktiven Einheit kombiniert sind, bezeichnet man diese auch als Eintankgenerator.

Zur Erzielung einer optimalen Bildqualität (Helligkeit, Kontrast, Signal-/Rauschverhältnis und Schärfe) des durchleuchteten Bereiches ist unter anderem die Intensität der auf den betreffenden Bereich einfallenden Röntgenstrahlung von entscheidender Bedeutung Im allgemeinen verbessert sich die Bildqualität mit steigender Röntgenstrahlendosis. Andererseits ist aber aus naheliegende Gründen darauf zu achten, dass der Patient nicht mit einer zu hohen Dosisleistung (Dosisrate) belastet wird. Dieser maximale Wert wird durch länderspezifische gesetzliche Regelungen begrenzt.

Die in den Patienten bzw. ein Objekt eintretende Dosisleistung ist im wesentlichen von der Einstellung der Spannung und des Stroms, mit der /dem die Röntgenröhre beaufschlagt wird, dem Abstand zwischen der Röntgenröhre und dem Objekt (SSD - source skin distance) sowie von Filtern abhängig, die gegebenenfalls in dem Strahlengang zwischen der Da diese Parameter während einer Untersuchung im allgemeinen durch eine Belichtungsautomatik geregelt bzw. durch den Anwender verändert werden können, sind besondere Vorkehrungen zu treffen, um zu verhindern, dass dabei die vorgegebene maximale, in den Patienten eintretende Dosisleistung überschritten wird.

Aus der EP 1 035 420 ist ein Verfahren und eine Vorrichtung zur Steuerung der Belichtung in radiologischen Bildsystemen bekannt, mit dem /der die Probleme gelöst werden sollen, die sich im Hinblick auf das Signal-/Rauschverhältnis ergeben, wenn bei einer geometrischen Vergrößerung eines darzustellenden Objektes durch Veränderung des Abstandes zwischen einer Röntgenstrahlenquelle und dem Objekt oder zwischen der Röntgenstrahlenquelle und einer Bildaufnahmeeinrichtung die Strahlendosis an dem Objekt konstant gehalten wird. Die Eintrittsdosis wird dort in Abhängigkeit von diesen beiden Abständen so verändert, dass eine geeignete äquivalente Dosis in der Objektebene konstant bleibt.

Eine allgemeine Aufgabe, die der Erfindung zugrunde liegt, besteht darin, ein Verfahren und eine Vorrichtung zu schaffen, mit der eine in ein Untersuchungsobjekt eintretende (Röntgen) Strahlendosisleistung (Eintritts-Dosisleistung) unabhängig von einer durch einen Anwender gewählten Regelkurve, mit der die Strahlenquelle betrieben wird, sowie unabhängig von Bestrahlungsbedingungen wie zum Beispiel dem Abstand zwischen dem Objekt und der Strahlenquelle, deren Strahlenausbeute sowie einem in den Strahlengang eingebrachten Filter, auf einen voreinstellbaren maximalen Wert begrenzt werden kann.

Weiterhin soll mit der Erfindung ein Verfahren und eine Vorrichtung zur Belichtung von Röntgenaufnahmen geschaffen werden, bei dem /der insbesondere bei Einsatz einer Belichtungsautomatik eine voreinstellbare maximale Eintritts-Dosisleistung unabhängig von der Regelkurve und den Bestrahlungsbedingungen nicht überschritten wird.

Es soll auch ein Verfahren und eine Vorrichtung zur Belichtung von Röntgenaufnahmen geschaffen werden, bei dem /der eine voreinstellbare maximale Eintritts-Dosisleistung unabhängig von den genannten Parametern nicht überschritten wird und das /die sowohl bei der Durchleuchtung (Fluoroskopie), als auch bei Aufnahmeserien (Einzelbilder) anwendbar ist.

Schließlich soll auch ein Röntgengenerator mit einer Vorrichtung zur Durchrührung eines der oben genannten Verfahren geschaffen werden.

Gelöst wird die Aufgabe gemäß Anspruch 1 mit einem Verfahren zur Begrenzung einer in ein Objekt eintretenden Strahlung (Eintritts-Dosisleistung) bei der Bestrahlung des Objektes mittels einer Strahlenquelle, insbesondere einer Röntgenstrahlenquelle, wobei die Strahlenquelle mit einer Regelkurve (I, II) angesteuert wird, die durch eine Mehrzahl von Wertepaaren gebildet ist, mit denen jeweils eine Zuordnung zwischen mindestens einem ersten und einem zweiten Betriebsparameter der Strahlenquelle vorgenommen wird, und wobei die Regelkurve in Abhängigkeit von Bestrahlungsbedingungen so korrigiert ist, dass bei der Bestrahlung die Eintritts-Dosisleistung für keines der Wertepaare überschritten wird.

Die Aufgabe wird gemäß Anspruch 6 mit einer Vorrichtung zur Durchführung des Verfahrens gelöst, die eine erste Speichereinrichtung für mindestens eine Regelkurve sowie mindestens eine Ausbeutekurve, eine programmierbare Rechnereinheit zur Berechnung der korrigierten Regelkurve, sowie eine zweite Speichereinrichtung zur Speicherung der korrigierten Regelkurve aufweist.

Ein besonderer Vorteil dieser Lösungen besteht darin, dass eine korrigierte Regelkurve zur Verfügung steht, die in voller Länge zur Einstellung gewünschter Röhrenparameter (Röhrenspannung, Röhrenstrom oder Ladung) und somit zur Optimierung der Bildqualität genutzt werden kann, ohne dass die Gefahr eines Überschreitens der maximalen Eintritts-Dosisleistung besteht.

Die Unteransprüche haben vorteilhafte Weiterbildungen der Erfindung zum Inhalt.

Die Ansprüche 2 und 3 beinhalten bevorzugte Regelkurven, während die Ansprüche 4 und 5 bevorzugte Bestrahlungsbedingungen bzw. die Art ihrer Berücksichtigung beschreiben.

Weitere Einzelheiten, Merkmale und Vorteile der Erfindung ergeben sich aus der folgenden Beschreibung von bevorzugten Ausführungsformen anhand der Zeichnung Es zeigt:
- Fig. 1: ein Blockschaltbild einer erfindungsgemäßen Röntgenanlage;
- Fig. 2: eine erste Regelkurve einer Röntgenröhre;
- Fig. 3: eine zweite Regelkurve einer Röntgenröhre;
- Fig. 4: eine Kurve der Ausbeute einer Röntgenröhre in Abhängigkeit von einer anliegende Spannung;
- Fig. 5: ein Flussdiagramm eines ersten erfindungsgemäßen Verfahrens; und
- Fig. 6: ein Flussdiagramm eines zweiten erfindungsgemäßen Verfahrens.

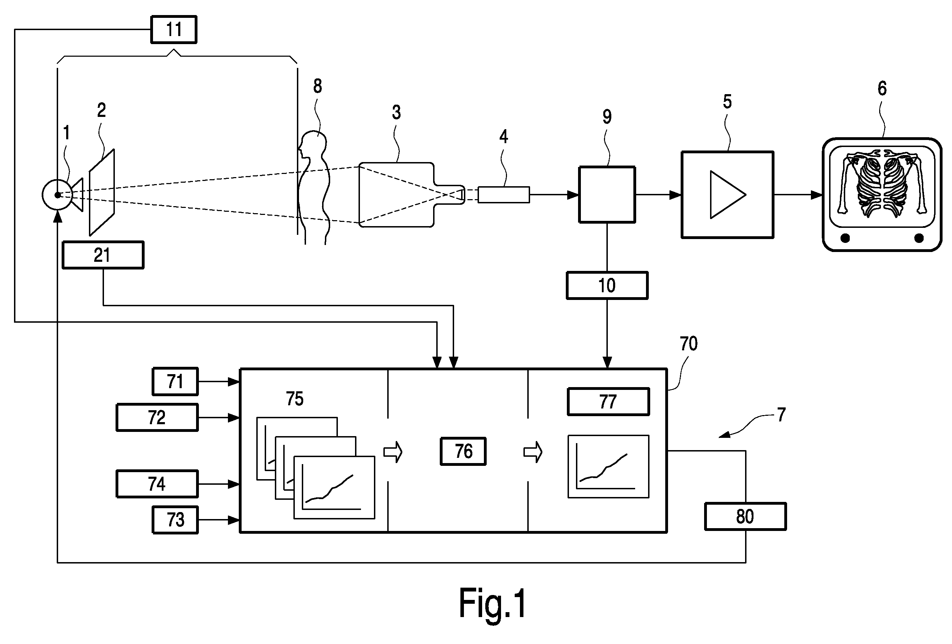

Figur 1 zeigt ein Blockschaltbild einer erfindungsgemäßen Röntgenanlage. Die Anlage umfasst eine Röntgenstrahlenquelle in Form einer Röntgenröhre 1, mit der Röntgenstrahlen auf ein Untersuchungsobjekt 8 (Patient) gerichtet werden. Der Abstand zwischen dem Brennpunkt der Röntgenröhre 1 und der Eintrittsfläche des zu untersuchenden Objektes (SSD - source skin distance) wird mit einer Entfernungsmesseinrichtung 11 erfasst. Zwischen der Röntgenröhre 1 und dem Untersuchungsobjekt 8 kann sich für bestimmte Untersuchungen ein Filter 2 befinden. Die Art des eingesetzten Filters wird mit einer Filtersensoreinrichtung 21 erfasst.

Die durch das Untersuchungsobjekt 8 hindurchgetretene Strahlung wird von einem Detektor 3, z. B. einem Sekundärelektronenvervielfacher (Bildverstärker) aufgenommen und von einer Kamera 4 in ein entsprechendes Bildsignal umgewandelt. Das Bildsignal wird anschließend über einen Strahlteiler mit Dosisleistungssensor 9 geführt, mit dem die durch das Objekt hindurchgetretene Dosisleistung erfasst und ein entsprechendes Dosisleistungssignal (10) erzeugt wird.

Das Bildsignal wird weiterhin mit einem Videoverstärker 5 aufbereitet und dann auf einem Monitor 6 zur Anzeige gebracht.

Zum Betrieb der Röntgenröhre 1 ist ein Röntgengenerator 7 vorgesehen, der sich im wesentlichen aus einer Steuereinrichtung 70 und einer durch diese angesteuerten Spannungsversorgung 80 für die Röntgenröhre 1 zusammensetzt, wobei die Steuereinheit 70 als Bestandteil des Röntgengenerators oder als separate Einheit ausgeführt sein kann.

An der Steuereinrichtung 70 liegen das mit der Entfernungsmesseinrichtung 11 erzeugte Entfernungssignal, das mit der Filter-Sensoreinrichtung 21 erzeugte Filtersignal sowie das mit dem Dosisleistungssensor 9 erzeugte Dosisleistungssignal (10) an.

Weiterhin können an der Steuereinrichtung 70 durch einen Benutzer verschiedene Einstellungen vorgenommen bzw. Vorgaben gemacht werden. Dies sind im wesentlichen die Auswahl (71) der für die betreffende Untersuchung geeigneten Regelkurve, die Einstellung (72) einer für die Untersuchung maximalen, nicht zu überschreitenden Dosisleistung an dem Untersuchungsobjekt (Eintritts-Dosisleistung), die Einstellung (73) der Startwerte von Röhrensparmung und /oder Röhrenstrom, von denen ausgehend eine Belichtungsautomatik die Aufnahmen belichtet, sowie im Falle von Aufnahmeserien (einzeln belichtete statische Aufnahmen) die Einstellung (74) einer Pulsfolgefrequenz für die Röhrenspannung.

Die Steuereinrichtung 70 beinhaltet im wesentlichen eine erste Speichereimrichtung 75 für eine Mehrzahl von Regelkurven zum Betrieb der Röntgenröhre. Die Regelkurven sind jeweils durch eine Mehrzahl von Wertepaaxen gebildet, mit denen jeweils eine Zuordnung zwischen einem ersten und einem zweiten Betriebsparameter der Röntgenxöhre vorgenommen wird, wie zum Beispiel Röhrenspannung und Röhrenstrom oder eine Ladung.

Mit der Anzahl von Regelkurven werden jeweils unterschiedliche Regelprofile realisiert, die von dem Benutzer des Systems entsprechend der Art der Untersuchung ausgewählt werden können, um eine optimale Bildqualität zu erzielen.

Weiterhin dient die erste Speichereinrichtung 75 zur Speicherung einer Mehrzahl von Ausbeutekurven, die jeweils durch eine Mehrzahl von Wertepaaren gebildet sind, mit denen jeweils eine Zuordnung zwischen der Anzahl von ersten oder zweiten Betriebsparametern und einer damit erzielbaren Dosisleistung, die auf einen Röhrenstrom sowie einen Abstand von der Röntgenröhre normiert ist, vorgenommen wird

Die Steuereinrichtung 70 beinhaltet ferner eine Rechnereinheit 76, mit der in Abhängigkeit von dem verwendeten Typ der Röntgenröhre sowie den zugeführten Signalen (insbesondere dem Filtersignal) und den vorgenommenen Einstellungsn eine entsprechende Ausbeutekurve ausgewählt und die durch den Benutzer ausgewählte Regelkurve rechnerisch korrigiert wird, sowie eine zweite Speichereinrichtung 77 zur Speicherung der korrigierten Regelkurve.

Die Belichtungsautomatik (nicht dargestellt) bestimmt in Abhängigkeit von dem von dem Dosisleistungssensor 9 erzeugten Dosisleistungssignal (10) den für eine optimale Belichtung geeigneten Wert des ersten Betriebsparameters. Anhand der korrigierten Regelkurve wird der diesem Wert zugeordnete Wert des zweiten Betriebsparameters bestimmt. Dieses Wertepaar wird dann der Spannungsversorgung 80 zugeführt.

Die Spannungsversorgung 80 ist schließlich mit der Röntgenröhre 1 verbunden und erzeugt entsprechend dem zugsführten Wertepaar die zum Betrieb der Röntgenröhre 1 erforderlichen Spannungen und Ströme, die im Falle von Aufnahmeserien geeignete zeitliche Verläufe (z. B. Rechteck-Pulsform) aufweisen.

Zur Durchführung einer Röntgenuntersuchung müssen die Röntgenröhre 1 und das Untersuchungsobjekt 8 relativ zueinander in eine zur Abbildung des zu untersuchenden Bereiches optimale Position verfahren werden.

Durch eine Relativbewegung entlang der Richtung des Röntgenstrahls erhöht oder vermindert sich die Dosisrate an der Eintrittsstelle des Untersuchungsobjektes. Bei einer Relativbewegung in einer Richtung senkrecht zu dem Röntgenstrahl tritt dieser im all gemeinen durch Bereiche des Untersuchungsobjektes mit unterschiedlichen Absorptionseigenschaften. Beide Bewegungsrichtungen haben somit eine Veränderung der Dosisrate an dem Bildverstärker 3 und somit auch eine Veränderung der Helligkeit des auf dem Monitor 6 wiedergegebenen Bildes zur Folge.

Da eine Verminderung der aufgenommenen Dosisrate zu einer Beeinträchtigung der Bildqualität führt, wird die Spannung und /oder der Strom, mit der /dem die Röntgenröhre 1 beaufschlagt wird, bei abnehmender Dosisrate automatisch mit der Belichtungsautomatik oder ggf. auch manuell entsprechend erhöht. Dabei ist allerdings auch zu berücksichtigen, dass eine zu starke Erhöhung der Dosisrate einerseits zu einer Überstrahlung von Einzelheiten in dem erzeugten Bild führen kann. Andererseits ist natürlich aus gesundheitlichen Gründen die Strahlenbelastung des Patienten möglichst gering zu halten, und es sollte eine vorgegebene maximale Eintritts-Dosisrate an dem Patienten nicht überschritten werden.

Dies ist insbesondere bei einer Veränderung des Abstandes zwischen der Röntgenröhre und dem Patienten oder dem Einsetzen eines anderen Filters in den Strahlengang zu beachten, da sich dadurch die Eintritts-Dosisleistung erheblich erhöhen kann.

Die Steuereinrichtung 70 ist so ausgelegt, dass die maximale Eintritts-Dosisrate über den gesamten Regelbereich der Belichtungsautomatik nicht überschritten wird Dies soll nun im Detail anhand eines Beispiels beschrieben werden.

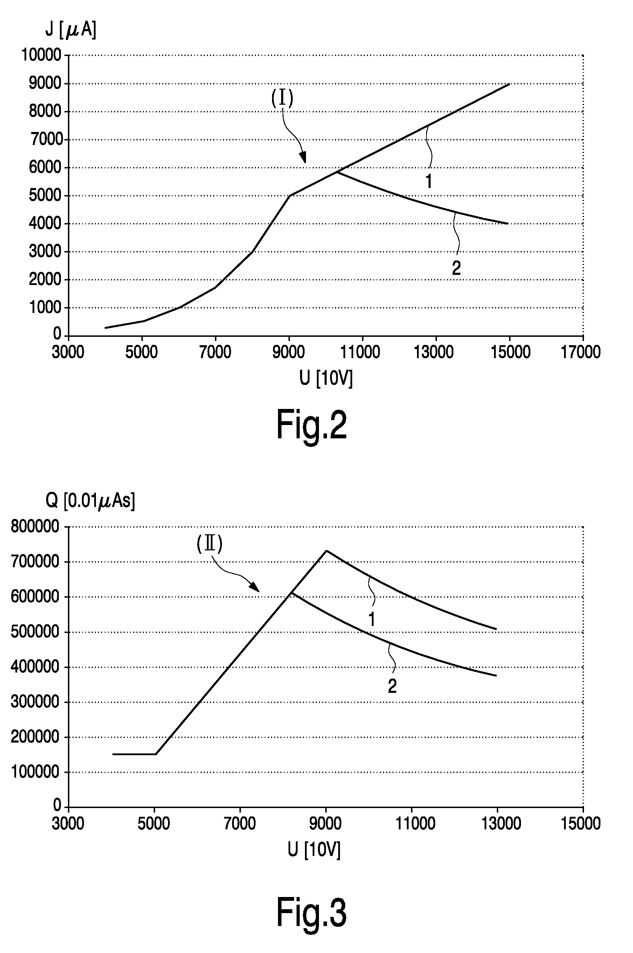

Im einzelnen ist - wie bereits oben angedeutet wurde - in der Steuereinrichtung 70 eine Mehrzahl von ersten Regelkurven für die Durchleuchtung gespeichert, mit denen jeweils verschiedenen Röhrenspannungen U die entsprechenden Röhrenströme I zugeordnet sind Weiterhin ist eine Mehrzahl von zweiten Regelkurven für Aufnahmeserien gespeichert, mit denen jeweils verschiedenen Röhrenspannungen U die entsprechenden Ladungswerte Q zugeordnet sind Figur 2 zeigt beispielhaft eine der ersten Regelkurven (I), während in Figur 3 eine der zweiten Regelkurven (II) dargestellt ist.

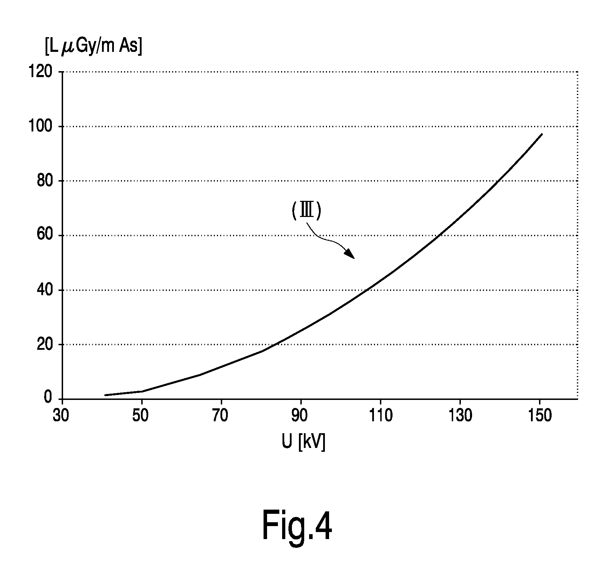

Die ebenfalls gespeicherten Ausbeutekurven (III), von denen eine in Figur 4 dargestellt ist, beschreiben jeweils den Zusammenhang zwischen der an die Röntgenröhre angelegten Röhrenspannung U und der damit erzielten Dosisrate Y pro mA des Röhrenstroms unter Berücksichtigung eines gegebenenfalls in den Strahlengang eingesetzten Filters, und zwar in einem auf einen Meter normierten Abstand von der Röntgenröhre (Ausbeute). Jeder Röntgenaufnahme wird somit die dem verwendeten Filter und der verwendeten Röntgenröhre zugeordnete Ausbeutekurve zugrunde gelegt.

Um zu verhindern, dass bei einer Veränderung eines oder mehrerer der durch den Benutzer einstellbaren bzw. vorgebbaren Parameter (insbesondere Abstand zwischen Röntgenröhre und Untersuchungsobjekt, Röhrenspannung, Röhrenstrom, Röhrentyp, Filtertyp, Pulsgeschwindigkeit) eine voreingestellte maximale Dosisrate der in das Objekt eintretenden Röntgenstrahlen überschritten wird, werden die Regelkurven unter Berücksichtigung dieser maximalen Eintritts-Dosisrate korrigiert.

Hierbei ist zu unterscheiden zwischen der Korrektur der ersten Regelkurven (I) für eine Durchleuchtung und der Korrektur der zweiten Regelkurven (II) für Aufnahmeserien.

Für eine Durchleuchtung wird die von dem Benutzer ausgewählte erste Regelkurve zunächst rechnerisch analysiert, um das in der Darstellung der Figur 2 rechte obere Ende der Kurve zu ermitteln, das heißt den Wert der höchsten Röhrenspannung sowie den diesem Spannungswert zugeordneten Wert des Röhrenstroms. Bei dem Beispiel in Figur 2 ergibt sich für eine maximale Spannung von 150 kV ein Strom von 9 mA.

Weiterhin wird anhand der entsprechend dem verwendeten Filter (und der Röntgenröhre) ausgewählten Ausbeutekurve ermittelt, wie hoch die Ausbeute für diese maximale Spannung ist. Bei dem in Figur 4 gezeigten Beispiel ergibt sich für eine maximale Spannung von 150 kV eine Ausbeute von etwa 96,4 µGy/mAs.

Durch Multiplikation dieser Ausbeute mit dem ermittelten maximalen Strom erhält man die maximale Dosisleistung, die sich in einem Abstand von einem Meter von der Röntgenröhre bei der maximalen Röhrenspannung ergeben würde. In dem genannten Beispiel beträgt diese Dosisleistung 867,6 µGy/s.

Anschließend wird durch Multiplikation dieses Wertes mit dem umgekehrten Quadrat des tatsächlichen Abstandes der Röntgenröhre von dem Untersuchungsobjekt ermittelt, wie hoch die maximale, auf das Untersuchungsobjekt einfallende Dosisleistung wäre. Bei einem mit der Entfernungsmesseinrichtung 11 ermittelten Abstand von 0,8 Metern ergibt sich für dieses Beispiel ein Wert von 1355,63 µG/s.

Dieser Wert wird nun mit dem voreingegebenen Wert der maximalen Eintritts-Dosisrate (zum Beispiel 600 µGy/s) verglichen. Durch Division dieses maximalen Wertes durch den berechneten Wert erhält man einen Reduktionsfaktor (in diesem Beispiel 0,4426), mit dem nun der der höchsten Röhrenspannung zugeordnete Stromwert (hier 9 mA) multipliziert wird Dadurch ergibt sich ein reduzierter Stromwert (hier 3,9834 mA) für die maximale Röhrenspannung und dementsprechend eine reduzierte maximale Leistung von 597,51 W.

Dieses Wertepaar (reduzierter Stromwert, maximale Spannung) wird nun als das in Richtung steigender Spannungen liegende obere (letzte) Wertepaar und damit als das Ende der korrigierten Regelkurve festgelegt. Ausgehend von diesem oberen Ende wird dann der Verlauf der Regelkurve so korrigiert, dass sie an keiner Stelle die berechnete reduzierte maximale Leistung und damit die vorgegebene maximale Eintritts-Dosisrate übersteigt.

Dies wird dadurch erreicht, dass ein neuer (oberer) Kurvenabschnitt (2) festgelegt wird, durch den Spannungswerte unterhalb des maximalen Spannungswertes jeweils reduzierten Stromwerten zugeordnet werden, deren Produkt jeweils eine Leistung ergibt, die gleich oder kleiner als die reduzierte maximale Leistung ist. Der neue Kurvenabschnitt (2) erstreckt sich somit in Richtung auf die ursprüngliche Regelkurve, bis er diese schneidet, und ersetzt damit den Teil (1) der ursprünglichen Regelkurve, der oberhalb des Schnittpunkts (hier bei 102,5 kV) dieser beiden Kurven liegt. Die korrigierte Regalkurve setzt sich somit aus einem ersten unteren Abschnitt, der dem ursprünglichen Verlauf entspricht, sowie dem neuen zweiten oberen Abschnitt (2) zusammen.

Der zweite obere Abschnitt kann natürlich auch so verlaufen, dass sich das Produkt aus Röhrenspannung und zugeordnetem Stromwert ausgehend von dem oberen Wertepaar in Richtung auf die ursprüngliche Kurve verkleinert, wobei der Schnittpunkt dann erst bei niedrigeren Spannungswerten erreicht wird

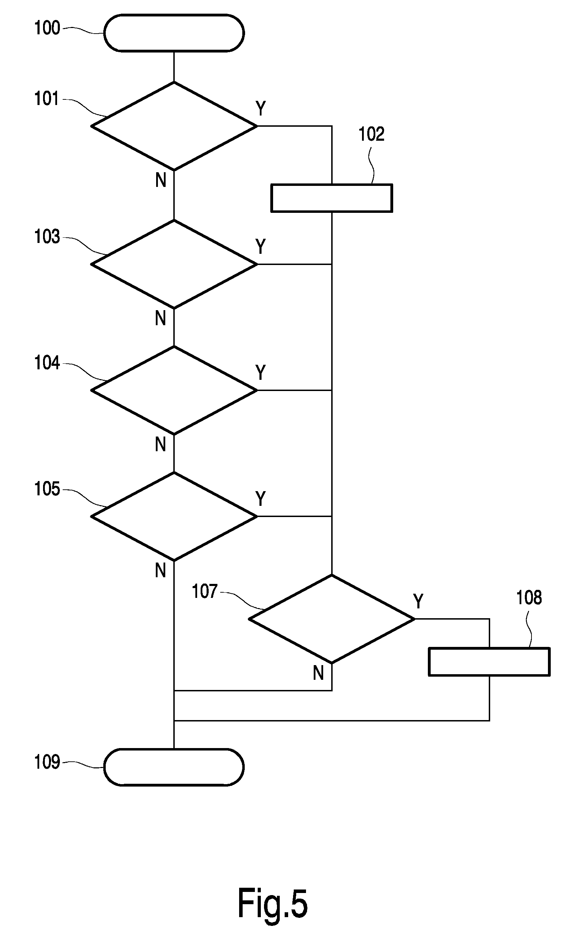

Das Verfahren wird vorzugsweise in Form eines auf der Rechnereinheit 76 ausgeführten Programms implementiert. Zur Verdeutlichung des Programmablaufes sei dieser anhand des Flussdiagramms in Figur 5 erläutert.

Nach dem Programmstart 100 wird mit einem eisten Schritt 101 zunächst abgefragt, ob der Filter 2 und /oder die Röntgenröhre 1 nach einer bereits zuvor durchgeführten Kurvenkorrektur geändert wurden. Wenn dies der Fall ist, wird gemäß einem zweiten Schritt 102 die dieser neuen Röhren/Filter-Kombination zugeordnete Ausbeutekurve ausgewählt und der Ablauf mit einem siebten Schritt 107 fortgesetzt.

Wenn die Abfrage mit dem ersten Schritt 101 verneint wird, wird mit einem dritten Schritt 103 abgefragt, ob seit der letzten Korrektur die Regelkurve geändert wurde. Wenn dies der Fall ist, wird mit dem siebten Schritt 107 fortgefahren. Andernfalls wird mit einem vierten Schritt 104 abgefragt, ob sich seit der letzten Korrektur der Abstand zwischen der Röntgenröhre und dem Untersuchungsobjekt 8 verändert hat. Wenn dies der Fall ist, wird mit dem siebten Schritt 107 fortgefahren, andernfalls wird mit einem fünften Schritt 105 abgefragt, ob die vorgegebene maximale Eintritts-Dosisleistung seit der letzten Korrektur geändert wurde. Wenn diese Abfrage mit Ja beantwortet wird, wird mit dem siebten Schritt 107 fortgefahren, andernfalls wird das Verfahren mit einem neunten Schritt 109 beendet.

Mit dem siebten Schritt 107 wird abgefragt, ob die erste Regelkurve gemäß Figur 2 Wertepaare enthält, mit denen die vorgegebene maximale Eintritts-Dosisleistung an dem Untersuchungsobjekt überschritten werden kann. Wenn dies der Fall ist, wird die Regelkurve mit einem achten Schritt 108 korrigiert und in der zweiten Speichereinrichtung 77 gespeichert. Das Programm wird dann, ebenso wie in dem Fall, in dem die Abfrage mit dem siebten Schritt 107 verneint wird, mit dem neunten Schritt 109 beendet.

Der siebte Schritt 107 und der achte Schritt 108 werden gemäß obiger Erläuterung im Zusammenhang mit der Korrektur der ersten Regelkurve ausgeführt.

Zur Korrektur der zweiten Regelkurve (Figur 3) für Aufnahmeserien, bei denen die Röntgenröhre gepulst betrieben wird, ist der vorgegebene maximale Wert der Eintritts-Dosisrate (zum Beispiel 800 µGy/s) durch die Anzahl von Belichtungsintervallen pro Sekunde (das heißt der Pulsfolgefrequenz, zum Beispiel 2/s) zu dividieren. Dadurch erhält man einen maximalen Dosiswert für einen einzelnen Puls (ein Belichtungsintervall), der der weiteren Betrachtung zugrunde gelegt wird und im hier betrachteten Beispiel 400 µGy beträgt.

Die Analyse der von dem Benutzer ausgewählten zweiten Regelkurve beginnt mit einer Ermittlung der Ladung, die sich für die maximale Röhrenspannung ergibt. Aus der in Figur 3 gezeigten Kurve ergibt sich für die maximale Spannung von 130 kV eine Ladung von etwa 5,17 mAs.

Weiterhin wird wiederum anhand der entsprechend dem verwendeten Filter ausgewählten Ausbeutekurve ermittelt, wie hoch die Ausbeute für die maximale Spannung ist. Bei dem in Figur 4 gezeigten Beispiel ergibt sich für eine maximale Spannung von 130 kV eine Ausbeute von etwa 67 µGy/mAs.

Durch Multiplikation dieser Ausbeute mit der ermittelten maximalen Ladung erhält man die maximale Dosis, die sich in einem Abstand von einem Meter von der Röntgenröhre bei der maximalen Röhrenspannung ergibt. In dem genannten Beispiel beträgt diese Dosis 346,4 µGy.

Anschließend wird durch Multiplikation dieses Wertes mit dem umgekehrten Quadrat des tatsächlichen Abstandes der Röntgenröhre von dem Untersuchungsobjekt ermittelt, wie

hoch die maximale, auf das Untersuchungsobjekt einfallende Dosis ist. Bei einem mit der Entfernungsmesseinrichtung 11 ermittelten Abstand von 0,8 Metern ergibt sich für dieses Beispiel ein Wert von 541,25 µGy.

Dieser Wert wird nun mit dem oben berechneten Wert der maximalen Dosis für einen Puls (hier 400 µGy) verglichen. Durch Division dieses maximalen Wertes durch den berechneten Wert erhält man einen Reduktionsfaktor (in diesem Beispiel 0,74), mit dem nun der mit der Regelkurve ermittelte Ladungswert (hier 5,17 mAs) multipliziert wird. Dadurch ergibt sich ein reduzierter Ladungswert (hier 3,827 mAs) für die maximale Röhrenspannung von 130 kV und dementsprechend eine reduzierte maximale Energie von 497,51 Ws.

Dieses Wertepaar (reduzierter Ladungswert, maximale Spannung) wird nun als das in Richtung steigender Spannungen liegende obere (letzte) Wertepaar und damit als das Ende der korrigierten Regelkurve festgelegt. Ausgehend von diesem oberen Ende wird dann der Verlauf der Regelkurve wiederum so korrigiert, dass an keiner Stelle die berechnete reduzierte maximale Energie und damit bei der gegebenen Pulsfolgefrequenz die vorgegebene maximale Eintritts-Dosisrate überschritten wird.

Dies wird dadurch erreicht, dass ein neuer (oberer) Kurvenabschnitt (2) festgelegt wird, durch den Spannungswerte unterhalb des maximalen Spannungswertes jeweils reduzierten Ladungswerten zugeordnet werden, deren Produkt jeweils eine Energie ergibt, die gleich oder kleiner als die maximale Energie ist. Der neue Kurvenabschnitt erstreckt sich somit in Richtung auf die ursprüngliche Regelkurve, bis er diese schneidet, und ersetzt damit den Teil (1) der ursprünglichen Regelkurve, der oberhalb des Schnittpunkts (hier bei 81,2 kV) dieser beiden Kurven liegt. Die korrigierte Regelkurve setzt sich somit wiederum aus einem ersten unteren Abschnitt, der dem ursprünglichen Verlauf entspricht, sowie dem neuen zwdten oberen Abschnitt (2) zusammen.

### Der zweite obere Abschnitt kann auch hierbei so verlaufen, dass sich das Produkt aus

Röhrenspannung und zugeordnetem Ladungswert ausgehend von dem oberen Wertepaar in Richtung auf die ursprüngliche Kurve verkleinert, wobei der Schnittpunkt dann erst bei niedrigeren Spannungswerten erreicht wird.

Auch dieses Verfahren wird vorzugsweise in Form eines auf der Rechnereinheit 76 ausgeführten Programms implementiert.

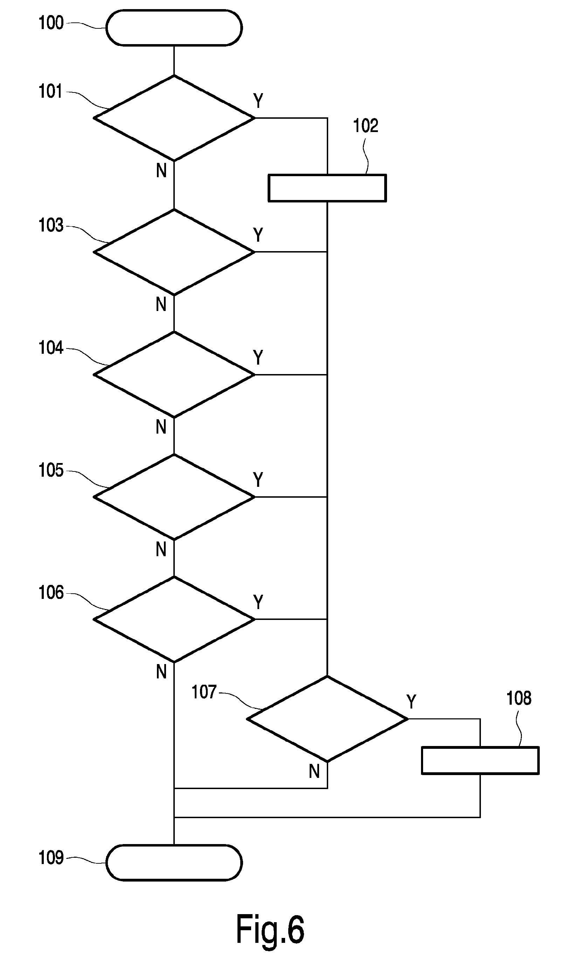

Die wesentlichen Schritte eines solchen Programms sind in Figur 6 dargestellt. Dieser Programmablauf unterscheidet sich von demjenigen gemäß Figur 5 dadurch, dass nach dem fünften Schritt 105 ein sechster Schritt 106 eingefügt ist, mit dem abgefragt wird, ob seit der letzten Korrektur die Pulsfolgefrequenz geändert wurde. Wenn dies der Fall ist, wird, ebenso wie bei einer positiven Beantwortung der Abfragen mit den Schritten 101 bis 105, der Ablauf mit dem siebten Schritt 107 fortgesetzt, mit dem abgefragt wird, ob die zweite Regelkurve gemäß Figur 3 Wertepaare enthält, mit denen die vorgegebene maximale Eintritts-Dosisleistung überschritten werden kann. Wenn diese Abfrage mit Ja beantwortet wird, wird die Regelkurve mit dem achten Schritt 108 korrigiert, und die korrigierte Regelkurve wird in der zweiten Speichereinrichtung 77 abgespeichert.

Der siebte Schritt 107 und der achte Schritt 108 werden gemäß obiger Erläuterung im Zusammenhang mit der Korrektur der zweiten Regelkurve ausgeführt.

Die Regelkurven konnten auch in der Weise korrigiert werden, dass nicht ein neuer zweiter oberer Kurvenabschnitt (2) ermittelt wird, sondern dass nur der erste untere Abschnitt verwendet wird, das heißt die korrigierte Regelkurve verläuft entsprechend der ursprünglichen Regelkurve nur bis zu dem Punkt, an dem das Wertepaar liegt, mit dem die maximale vorgegebene Eintritts-Dosisrate erreicht wird. Einer der besonderen Vorteile der Erfindung besteht jedoch darin, dass dies - von ganz wenigen Ausnahmen abgesehen - gerade nicht erforderlich ist.

Alternativ zu den in den Figuren 2 und 3 gezeigten Kurven können auch andere Regalkurven verwendet werden.

Eine Alternative sind zum Beispiel solche Regelkurven, bei denen die in den Figuren 2 und 3 jeweils auf der x- bzw. der y-Achse aufgetragenen ersten bzw. zweiten Betriebsparameter vertauscht sind.

Weiterhin ist es zum Beispiel möglich, den ersten Betriebsparameter in Form einer Pulsbreite der Röhrenspannung oder des Röhrenstroms zu verändern, wenn die Röntgenröhre gepulst betrieben wird. Die maximale Eintritts-Dosisleistung korrespondiert dabei mit einer maximalen Pulsbreite, die nicht überschritten werden darf.

Eine weitere Alternative besteht auch darin, eine dreidimensionale Regelkurve mit drei Betriebsparametern festzulegen, wobei ein Betriebsparameter eingestellt und die Regelkurve durch die oben beschriebene Berechnung eines oder beider anderen Betriebsparameter korrigiert wird. Dies können zum Beispiel eine Pulsfolgefrequenz sowie die Röhrenspannung und der Röhrenstrom sein.

Das erfindungsgemäße Prinzip lässt sich auf diese Weise auf nahezu jede Kombination von zwei oder mehr Betriebsparametern ausdehnen, wobei diese Parameter in erster Linie die Röhrenspannung, der Röhrenstrom, die Pulsbreite dieser Größen im gepulsten Betrieb und die Pulsfolgefrequenz dieser Größen sind Denkbar wäre darüber hinaus auch eine Veränderung des Abstandes zwischen der Röntgenröhre und dem Untersuchungsobjekt sowie ein Austausch von Filtern mit unterschiedlicher Strahlenabsorption.

Das erfindungsgemäße Verfahren ist schließlich auch für fotografische Einzelaufnahmen anwendbar.

## Patentansprüche

1. Verfahren zur Begrenzung einer in ein Objekt eintretenden Strahlung (Eintritts-Dosisleistung), bei der Bestrahlung des Objektes mittels einer Strahlenquelle, insbesondere einer Röntgenstrahlenquelle, wobei die Strahlenquelle mit einer Regelkurve (I, II) angesteuert wird, die durch eine Mehrzahl von Wertepaaren gebildet ist, mit denen jeweils eine Zuordnung zwischen mindestens einem ersten und einem zweiten Betriebsparameter der Strahlenquelle vorgenommen wird, und wobei die Regelkurve in Abhängigkeit von Bestrahlungsbedingungen so korrigiert ist, dass bei der Bestrahlung die Eintritts-Dosisleistung für keines der Wertepaare überschritten wird.

2. Verfahren nach Anspruch 1,
bei dem der erste Betriebsparameter eine Röntgenröhren-Spannung und der zweite Betriebsparameter ein Röntgenröhren-Strom ist.

3. Verfahren nach Anspruch 1,
bei dem der erste Betriebsparameter eine Röntgenröhren-Spannung und der zweite Betriebsparameter das Produkt aus einem Röntgenröhren-Strom und der Belichtungszeit ist.

4. Verfahren nach Anspruch 1,
bei dem die Bestrahlungsbedingungen durch den Abstand zwischen dem Objekt und der Strahlenquelle, deren Strahlenausbeute sowie ein in den Strahlengang eingebrachtes Filter definiert sind.

5. Verfahren nach Anspruch 1,
bei dem die Bestrahlungsbedingungen in Form einer Ausbeutekurve (III) aus einer Mehrzahl von Wertepaaren definiert sind, mit denen jeweils eine Zuordnung zwischen einem der Betriebsparameter und einer normierten Röntgenstrahlung vorgenommen wird.

6. Vorrichtung zur Durchführung des Verfahrens nach einem der vorhergehenden Ansprüche, mit einer ersten Speichereinrichtung (75) für mindestens eine Regelkurve sowie mindestens eine Ausbeutekurve, eine programmierbare Rechnereinheit (76) zur Berechnung der korrigierten Regelkurve nach einem der Ansprüche 1 bis 5, sowie eine zweite Speichereinrichtung (77) zur Speicherung der korrigierten Regelkurve.

7. Verfahren zur Belichtung von Röntgenaufnahmen unter Anwendung eines Verfahrens nach einem der Ansprüche 1 bis 5.

8. Vorrichtung zur Belichtung von Röntgenaufnahmen,
mit einer Vorrichtung nach Anspruch 6 sowie einer Belichtungsautomatik, mit der anhand einer erfassten, das Objekt beaufschlagenden Dosisleistung und der korrigierten Regelkurve ein eine Spannungsversorgung (80) für die Röntgenröhre (1) ansteuerndes Signal erzeugt wird.

9. Röntgengenerator mit einer Spannungsversorgung (80) für eine Röntgenröhre (1) sowie einer Vorrichtung nach Anspruch 8.

10. Röntgenanlage mit einem Röntgengenerator nach Anspruch 9.
